Europäisches Patentamt

European Patent Office

Office européen des brevets

(19)

(11) Publication number: **0 326 395**
**A2**

## (12) EUROPEAN PATENT APPLICATION

(21) Application number: **89300779.9**

(22) Date of filing: **27.01.89**

(51) Int. Cl.[4]: **C 12 Q 1/70**
**C 12 N 9/00**

(30) Priority: **29.01.88 US 149834**

(43) Date of publication of application:
**02.08.89 Bulletin 89/31**

(84) Designated Contracting States: **DE FR GB**

(71) Applicant: **CITY OF HOPE**
**1500 East Duarte Road**
**Duarte California 91010-0269 (US)**

(72) Inventor: **Cantin, Edouard Maxime**
**1160 Colorado Boulevard**
**Los Angeles California 90041 (US)**

**Openshaw, Harry Ogden**
**611 Devon Place**
**Long Beach California 90807 (US)**

(74) Representative: **Hallybone, Huw George et al**
**CARPMAELS AND RANSFORD 43 Bloomsbury Square**
**London WC1A 2RA (GB)**

(54) Method of detecting and identifying certain viral sequences.

(57) Methods for detecting viral sequences associated with multiple sclerosis by amplifying viral sequences and hybridizing probes complementary to at least a portion of the amplification product, including a method of detecting any known human retrovirus by means of a single pair of amplification oligonucleotide primers and subsequently using separate oligonucleotide probes that hybridize to at least a portion of the amplification products of known human retroviruses.

EP 0 326 395 A2

Description

## METHOD OF DETECTING AND IDENTIFYING CERTAIN VIRAL SEQUENCES

This invention relates to methods for detecting and identifying certain viral sequences. In particular this invention relates to methods for detecting and identifying viral sequences in the tissue and blood of patients afflicted with multiple sclerosis (MS). A further aspect of this invention permits the detection of the presence of any human retrovirus in a human tissue or blood sample and the subsequent identification of the particular human retrovirus.

It has recently been shown by Saiki, R. K., et al., Science 2301350-1354 (1985) that small amounts of DNA samples, undetectable with standard nucleic acid hybridization methods, can be specifically detected after amplification. This method makes use of repeated synthesis of target nucleic acid sequences flanked by oppositely oriented primers, and is referred to as the polymerase chain reaction, or PCR. The PCR was initially used to amplify the β-globin genomic sequence for the prenatal diagnosis of sickle cell anemia. Id.; Mullis, K., et al., Methods in Enzymology 155:335-350 (1987).

The PCR method has been modified to amplify specific RNA target sequences. This modified method is disclosed in Murakawa, et al., Serial No. 941,379, filed December 15, 1986 and its continuation-in-part Serial No.   filed January 12, 1988. (and at DNA 74: 287 to 297 (1988)) The entirety of the specifications and claims of each of these applications as filed is incorporated herein and made a part hereof by express reference. This method is referred to hereinafter as the Murakawa technique.

In general, the Murakawa technique uses reverse transcriptase in the initial amplification cycles to produce DNA transcripts. An advantage of amplifying an RNA template rather than a DNA template is greater resolution because expression of the messenger RNA (mRNA) is higher eukaryotic cells is selective and often tissue specific or time dependent. Therefore amplification of RNA can rely on a less complex template. Amplified fragments can be sequenced to verify the specificity of the amplification.

According to the present invention, viral sequences of human viruses that may play a role in multiple sclerosis (MS) are amplified and detected. There is evidence that exposure to an unknown environmental agent sets the stage for MS because of the occurence of point epidemics of MS. Kurtzke, J.F., et al., Ann. Neurol. 5:6-21 (1979); Kurtzke, J.F., et al., Neurol. 32:143-150 (1982). Many studies have attempted to implicate viruses as this environmental agent in MS by use of immunological assays, electron microscopy, standard viral isolation or transmission, DNA reassociation kinetics, and in situ hybridization for viral genes or transcripts. Johnson, R., Advan. Neurol. 13:1-46 (1975) and Cook, S., et al., Neurol. 30(2):80-91 (1980) provide reviews of such studies.

Particular viruses that have been implicated as a cause for MS are measles, canine distemper, parainfluenza, herpes simplex (HSV), corona virus, and a human retrovirus, but there has been no consistent verification of any of these viruses play a role in the disease. Multiple agents, including different viruses, may also be involved as one aspect of the pathogenesis of the complex disease process. The virus or virus fragments likely remain in the central nervous system or in peripheral blood leukocytes long after exposure.

Of particular interest with respect to the methods of the present invention are HSV, human retroviruses, and corona viruses.

Koprowski, H., et al., Perspect. Biol. Med. 22:10-18 (1978) and Martin, J.R., Lancet 11:777-781 (1981) report that HSV may play a role in MS. Fraser, N.W., et al., Proc. Natl. Acad. Sci. USA 78:6461-6465 (1981) reported detection of HSV DNA in human brain tissue, thereby rendering possible in situ reactivation of the viruses in the human central nervous system. Of particular interest in the present invention are sequences of the herpes simplex virus-1 (HSV-1) IE₁ (ICPO) gene and gene transcript of HSV-1. The IE₁ gene was chosen because of reports by Stevens, J.G., et al., Science 235:1056-1059 (1987); Puga, A., et al., J. Virol. 61:1700-1703 (1987); and Croen, J., et al., New Eng. J. Med. 317:1427-31 (1987) of an anti-sense transcript in the area of the IE₁ gene in latently infected ganglia of mice and men.

With respect to the family of corona viruses, reports by Tanaka, R., et al., J. Neurol. Sci. 28:121-126 (1976), Burks, J.S., et al., Science 209:933-934 (1980), and Murray, R., et al., Neurol. 37(s):109 (1987) support the existence of corona virus with MS.

Several studies such as that of Koprowski, H., et al., Nature 318:154-160 (1985) have also suggested an association between MS and human retroviral infection.

According to the present invention, sequences from an unknown human retrovirus can be amplified, preferably by the Murakawa technique, using one pair of oligonucleotide primers and then identified by using know synthetic oligonucleotide probes or sequencing. Such amplification is possible only to the extent that the oligonucleotide primers used are complementary to sequences that are conserved among the known human retroviruses. It is known, for example, that there are conserved sequences in the DNA polymerase genes of a number of DNA viruses as reported by Larder, B., et al., J. Eur. Mol. Biol. 6:169-175 (1987) and Bernard, A., et al., J. Eur. Mol. Biol. Org. 6:4219-4225 (1987), and that there are also areas of conserved sequences in RNA dependent polymerases from plant, animal, and bacterial viruses as reported by Kamer, G., et al., Nucl. Acids Res. 12:7269-7282 (1984). Areas of homology have been reported by Chiu, I.M., et al., Science 223:364-370 (1984); Stephens, R., et al. Science 231:589-594 (1986); Toh, H., et al., Nature 305:827-829 (1983) Chiu, I.M., et al., Nature 317:366-368 (1985); and Johnson, M.S., et al., Proc. Natl. Acad. Sci. USA 83:7648-7652 (1986) in the reverse transcriptase gene of retroviruses.

## SUMMARY OF THE INVENTION

In general, the invention features methods of detecting and identifying human viral sequences associated with multiple sclerosis, in a blood or tissue sample, comprising amplifying a portion of RNA or DNA present in said sample; hybridizing an internal oligonucleotide probe complementary to at least a portion of the amplification product; and identifying the human viral sequence present in said sample by hybridization with at least one internal probe known to be complementary to at least a portion of the amplification product.

This invention further features methods of detecting and identifying a human retrovirus in a human blood or tissue sample, comprising amplifying a portion of the human retrovirus reverse transcriptase RNA present in said sample; hybridizing an internal oligonucleotide probe complementary to at least a portion of the amplification product; and identifying the human retrovirus present in said sample by hybridization with at least one internal probe known to be complementary to at least a portion of the amplification products of known human retroviruses. In preferred embodiments a single set of amplification oligonucleotide primers based on conserved nucleotide sequences of the human retroviruses is used to amplify the known human retroviruses and internal probes for each retrovirus are used to identify the particular human retrovirus.

Other advantages and features of the invention will be apparent from the following description of the preferred embodiments thereof, and from the claims.

## DESCRIPTION OF THE DRAWINGS

Fig. 1 shows a photograph of an ethidium bromide stained agarose gel containing amplified HSV-1 $IE_1$ (ICPO) gene transcripts.

Fig. 2 shows a photograph of an autoradiogram of amplified HSV-1 $IE_1$ (ICPO) gene transcripts hybridized with a $^{32}P$ labelled oligonucleotide probe designed to hybridize to sequences in the amplified product.

Fig. 3 shows a photograph of an ethidium bromide stained agarose gel containing amplified HSV-1 $IE_1$ (ICPO) gene sequences.

Fig. 4 shows a photograph of an autoradiogram of amplified HSV-1 $IE_1$ (ICPO) gene sequences hybridized with a $^{32}P$ labelled oligonucleotide probe designed to hybridize to sequences in the amplified product.

## DESCRIPTION OF THE PREFERRED EMBODIMENT

According to the methods of the present invention viral sequences associated with MS can be detected and identified. Further, via the methods of the present invention, a previously uncharacterized human retrovirus in a human blood or tissue sample can be detected, and then specifically identified.

With respect to MS, the present invention provides a qualitative and semi-quantitative test for detecting viral sequences from among HSV-1 and the human retroviruses HIV, HTLV-I, and HTLV-II contained in human blood or tissue samples. The present invention also provides a method for first detecting the presence of a human retrovirus with a single pair of oligonucleotide primers and then identifying the particular human retrovirus by using oligonucleotide probes specially designed to hybridize to certain sequences of each of the amplification products of the three known human retroviruses.

In order to detect human retroviral sequences using a single set of primers via the preferable Murakawa technique, areas of nucleotide sequence conservation among the reverse transcriptase genes of HTLV-I, HTLV-II, and HIV were determined. The search for areas of nucleotide sequence conservation was focused on retroviral pol genes and after analysis of retrovirus sequence data as reported by Chiu, I.M., et al., Science 223:364-370 (1984) and Johnson, M.S., et al., Proc. Natl. Aca. Sci. USA 83:7648-7652 (1986), two regions of highly conserved sequence homology in the amino terminal segment of the retroviral pol gene were identified. These two regions of the pol gene comprise five amino acids each and are separated by 30 amino acids as shown below:

Val Leu Pro Gln Gly--30 amino acids--Gln Tyr Met Asp Asp

Val = valine, Leu = leucine, Pro = proline, Gln = glutamine, Gly = glycine, Tyr = tyrosine, Met = methionine, Asp = aspartic acid.

Using the nucleotide sequence data of LAV/HIV as reported by Wain-Hobson, S., et al., Cell 40:9-17 (1985), two oligonucleotide primers based on this conserved amino acid sequence were designed for use with the Murakawa technique. Bam H1 and Hind III restriction endonuclease recognition sequences (linker sequences ) are appended to the 5' end of the two primers (primer 1 and primer 2), respectively, for both HSV-1 $IE_1$ and human retrovirus reverse transcriptase to facilitate cloning of the amplified product.

By using the two human retrovirus reverse transcriptase primers which are based on the two highly conserved sequences of the retroviral pol gene of the three recognized retroviruses of HIV, HTLV-I, and

3

HTLV-II, the Murakawa technique can amplify all three human retroviruses regardless of the RNA template derived from patient blood and tissue samples. Because the 30 amino acid sequence which is flanked by the two constant five-amino acid segments varies depending upon which of the three retroviruses is used as a template for amplification, three internal probe oligonucleotides which are specific for a portion of the amplification products for HIV, HTLV-I, or HTLV-II were designed. Each of the three probes hybridizes to at least a portion of the amplified sequence bracketed by the priming oligonucleotides.

The murakawa technique is performed using both RNA and DNA templates and the thermostable DNA polymerase (Taq) isolated from Thermus aquaticus as reported by Chien, A., et al., J. Bacteriol. 127:1550-1557 (1986). An advantage of using the Taq enzyme is cost, since the enzyme is heat stable and, unlike E. coli Klenow DNA polymerase, is not inactivated during the denaturing step. Further, because the polymerization reaction proceeds at a relatively high temperature (74°C), the specificity of the amplification is increased as compared to the specificity resulting from polymerization reactions using E. coli Klenow DNA polymerase at 37°C. In cases where lower polymerization temperatures have to be used, the polymerization reaction is carried out for approximately 5 minutes.

For amplification of a portion of the HSV-1 $IE_1$ (ICPO) gene using preferably the Murakawa technique, preferred primers are oligonucleotides with the following sequences:

```
Primer 1:

        Bam H1
    recognition site
                 |
       ┌─────────┴─────────┐
  5'-G G A T C C G G G C G T G G A G G G T G G G C A C-3'
             └2966
                                (encoding nucleotides 2966-2986)


Primer 2:

        Hind III
    recognition site
                 |
       ┌─────────┴─────────┐
  5'-A A G C T T C C C T G G C C G C G C C C C C C C G G C C C-3'
             └3201
                          (encoding nucleotides 3201-3179)
```

Use of primers 1 and 2 are described above yields an amplified fragment comprising 245 base pairs (nucleotides 2966-3201) which includes 10 base pairs from the linker sequences.

A preferred internal probe includes the following sequence:

```
5'-C T G G C C C G A C C C G C G C C T C T T C C T C-3'.
          (3094-3070)
```

The oligonucleotide primers for human retrovirus reverse transcriptase with the following preferred sequences are preferably used in the Murakawa technique to amplify specific sequences of the three human retrovirus reverse transcriptases; the base triplets of each of the two primers are labeled with the above-identified amino acid segments conserved among the human retroviral pol genes:

Primer 1:

```
        Bam H1
     recognition site
                |
      ┌────────────┐  ┌ Val ┌ Leu ┌ Pro ┌ Gln ┌ Gly ┌
  5'-G G A T C C │G T G│C T T│C C A│C A G│G G A│-3'
                 └2417
                    (encoding nucleotides 2417-2431)
```

Primer 2:

```
     Hind III
   recognition site
              |
    ┌──────────┐  ┌ Asp ┌ Asp ┌ Met ┌ Tyr ┌ Gln ┌
  5'-A A G C T T│A T C│A T C│C A T│G T A│T T G│-3'.
               └2533
                    (encoding nucleotides 2533-2519)
```

Use of primers 1 and 2 yields an amplified fragment comprising 128 base pairs (nucleotides 2417-2533) which includes 12 base pairs from the linker sequences.

A prefered internal probe for hybridization with a portion of the amplification product flanked by the priming oligonucleotides for each known human retrovirus includes the following sequences:

HIV      5'- G G A T C A C C A G C A A T A T T C C A-3'
                (2438-2457)

HTLV-I   5'- A A T A G T C C C A C C C T G T T C G A-3'
                (2970-2989)

HTLV-II  5'- A A C A G C C C C A C C C T C T T C G A-3'.
                (2968-2987)

Oligonucleotide internal probes used in accordance with the present invention are labelled at their 5' ends using adenosine 5'-[$\gamma^{32}$P] triphosphate ($\gamma^{32}$P-ATP) and T4 polynucleotide kinase as described by Murakami, A., et al., Biochem. 24:4041-4046 (1985).

## Example 1

DNA and RNA which is to be used as the templates that are to be amplified is extracted from nervous system tissue by standard methodology used in the laboratory as described by Puga, A., et al., Virology 89:102-111 (1978). One-5 μg sample of total cellular DNA or RNA is used in each amplification procedure. Amplification is performed in amplification buffer (10 mM tris-HC1, pH 8.3; 1.5 mM $MgCl_2$; 50 mM KC1; 1 mM dithiothreitol; and 10% DMSO), a large molar excess (1.5 mM) of each of the four deoxynucleoside triphosphates (dNTP), and 100 μm of each of the oligonucleotide primers in a final reaction volume of 100 μl. Magnesium concentration is optimized for each template to be amplified.

For the first amplification cycle, DNA or RNA samples are heated to 95°C for two minutes, centrifuged for 5 seconds, and then cooled to 37°C for two minutes to anneal template to primer. In cases where RNA template is to be amplified, the first amplification cycle comprises adding 1 μl of reverse transcriptase (2.0 units, BioRad) diluted in amplification buffer to the reaction mixture for a two minute incubation period in order to extend the complementary primer annealed to the viral RNA template. Subsequent amplification cycles are carried out be reheating the reaction mixture to 95°C for two minutes and repeating the cycle sequence. For cycles 2-5 both reverse transcriptase and Thermus aquaticus DNA Polymerase (New England Biolabs) are added for elongation of the primers. In cycle 6, RNase A is added (0.45 μg) to reduce the complexity of the RNA and to facilitate primer hybridization. For subsequent amplification cycles only the DNA polymerase is added at 1 unit per 10 cycles. For amplification of DNA template, reverse transcriptase and RNase A are not used in the amplification procedure, but otherwise the procedure described above for RNA template amplification is the same.

After completion of the last cycle of amplification, preferably about 20 cycles, the resulting amplification products are placed on ice and 10-20 μl portions are electrophoresed in a 1.8% agarose gel. The amplified DNA is transferred to Zetaprobe (BioRad) using the alkaline blotting procedure of Harper, M., et al., Proc. Natl. Acad. Sci. USA 83:772-776 (1986) and prehybridized. Prehybridization reactions are performed at 65°C for 1-3 hours in 20 ml of 6X SSPE (1.0 M NaCl, 0.06 M NaPO$_4$, 0.006 M EDTA); 1.0% SDS; 0.5% rehydrated, powdered skim milk [(Alba) "blotto"]; and 10 μg per ml of sonicated, denatured herring sperm DNA. The hybridization reactions are carried out in 20 ml of the same buffer except the herring sperm DNA is replaced with 20 pmole of a 5'-$^{32}$P-labelled oligonucleotide probe (about 3 X 10$^8$ cpm). Hybridizations are performed for 1 to several hours at 65°C. In all cases, the hybridized membranes are washed with three 250 ml volumes of 6X SSC (0.95 M NaCl, 0.095 M Na citrate) and 0.1% SDS at 65°C for 5 minutes each, and autoradiographed at -70°C on Kodak XAR-5 film with an intensifying screen. Under non-denaturing electrophoresis conditions, two bands of hybridization are seen from the autoradiogram: an upper band of double-stranded amplified DNA and a lower band of single-stranded amplified DNA.

## Example 2

HSV-1 IE$_1$ gene transcripts were amplified by the methods described in Example 1 which were modified such that sense or anti-sense transcripts of the gene fragment contained in the samples were selectively amplified.

The oligonucleotide primers and internal probe for HSV-1 IE$_1$ as set forth above were used. However, in order to selectively amplify sense or anti-sense transcripts, only one primer was added during the first three reverse transcriptase cycles. In subsequent cycles, the second primer was added to the reaction mixture along with Thermus aquaticus DNA polymerase.

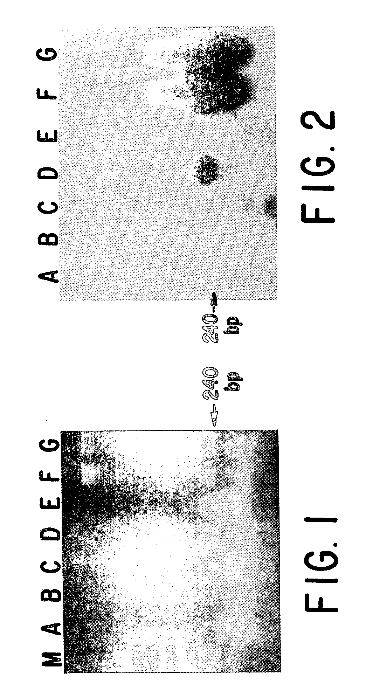

Fig. 1 shows a photograph of an ethidium bromide stained agarose gel of the amplification products obtained. The primers were designed to amplify a 235 base pair fragment, excluding linker sequences. Lane C contains the amplification product from a 6.5 ng sample of RNA template from infected vero cells. Primer 1 was used in the first three reverse transcriptase cycles and then primer 2 was added in order to amplify anti-sense RNA transcripts. Lane D contains the amplification product from the same material as used in Lane C except that primer 2 was used in the first three reverse transcriptase cycles and then primer 1 was added in order to amplify sense RNA transcripts. Lane E contains the amplification product from a 6.5 ng sample of RNA template from uninfected vero cells. Both primers 1 and 2 were used in all the amplification cycles in order to amplify both sense and anti-sense RNA transcripts. Lanes F and G contain amplification products from respectively, 470 ng and 270 ng samples of plasmid DNA containing the HSV-1 IE$_1$ gene.

The expected approximately 245 base pair amplified band is seen most clearly only in Lanes D, F and G. In Lanes C and E a slightly smaller band is visible.

The gel was then transferred to a Zetaprobe nylon membrane using the alkaline blotting technique, prehybridized as described in Example 1, and hybridized with the $^{32}$P-labelled oligonucleotide internal probe described above for HSV-1 IE$_1$. The hybridized membranes were washed stringently with 6X SSC and autoradiographed for one hour at -70°C on Kodak XAR-5 film with two Quanta III intensifying screens. A photograph of the resulting autoradiogram is shown in Fig. 2. As seen in Fig. 2, only the approximately 245 base pair amplified band in Lanes D, F, and G hybridized to the probe, whereas the slightly smaller bands in Lanes C and E did not hybridize to the probe. Thus, the sense transcript from a starting sample of 6.5 ng of RNA was successfully detected and identified.

## Example 3

HSV-1 IE$_1$ gene sequences were amplified by the methods described in Example 1 for DNA templates. DNA was extracted from ten pooled mouse trigeminal ganglia obtained from uninfected, acutely, and latently infected mice as described by Puga, A., et al., J. Virol. 89:102-111 (1978). Amplification primers 1 and 2 set forth above for the HSV-1 IE$_1$ gene were used.

In the first amplification cycle, all the samples were denatured at 98°C for 10 minutes and then cooled to room temperature for 5 minutes. 3 units of Thermus aquaticus polymerase were added to each sample, mixed, and centrifuged. Each sample was then overlayed with approximately 20 μl of mineral oil and then incubated at 70°C for 5 minutes for polymerization.

In subsequent cycles, the reaction mixtures were heated to 98°C for 1 minute to denature the DNA strands, cooled to 37°C for 1 minute, and then heated to 70°C for 5 minutes for polymerization. After 30 amplification cycles were completed for each sample, the samples were stored overnight at -20°C. A 20 µl aliquot of each reaction product was then electrophoresed in a 2% agarose gel in tris-borate EDTA. The gel was then stained with ethidium bromide and photographed.

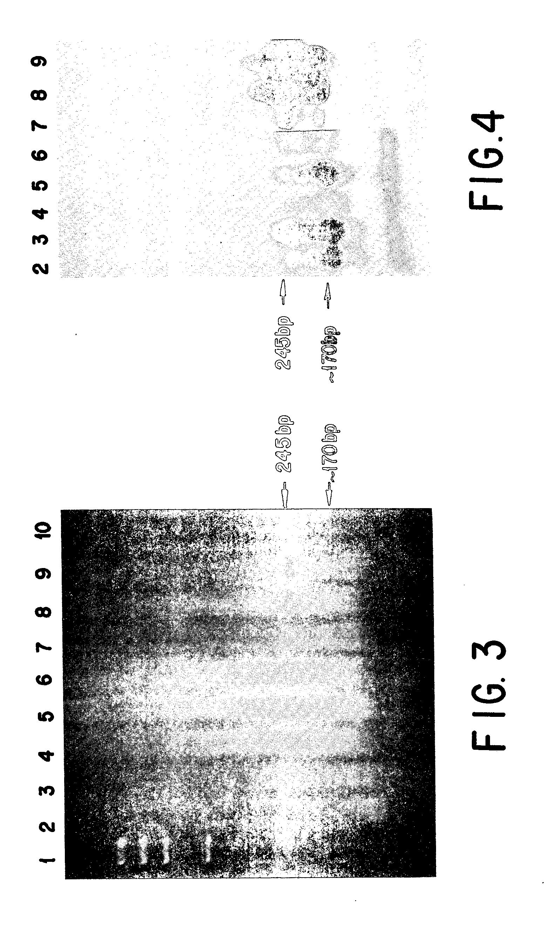

Fig. 3 shows the photograph resulting from the above-described procedures. Lane 1 contains ΦX174 Hae III molecular weight markers; Lane 2 contains 100 ng of trigeminal ganglion DNA-acute stage of HSV infection; Lane 3 contains 1 µg of the same DNA as in Lane 2; Lane 4 contains 1 µg of trigeminal ganglion DNA-latent stage of HSV infection; Lane 5 contains 5 µg of the same DNA as in Lane 4; Lane 6 contains 0.01 ng Bam-B fragment which contains the IE$_1$ gene coding sequence; Lane 7 contains 0.1 ng of Bam-B fragment; Lane 8 contains 50 ng of Bam-B fragment; Lane 9 contains 1 µg of HSV-1 (strain F) DNA; and Lane 10 contains lambda, EcoRI, and Hind III molecular weight markers.

The DNA was next transferred to a Zetaprobe nylon membrane using the alkaline blotting technique, prehybridized as described in Example 1, and hybridized with $^{32}$P-labelled oligonucleotide internal probe as described above for the HSV-1 IE$_1$ gene. The hybridized membranes were washed stringently with 6X SSC prior to autoradiography for one hour with two Quanta III intensifying screens at -70°C. The resulting autoradiogram is shown in Fig. 4 with molecular marker-containing Lanes 1 and 10 removed.

The expected specific amplified band of approximately 245 base pairs is most visible in Lanes 8 and 9 of the Fig. 3 ethidium bromide stained gel, although other lanes show some of the expected amplification bands also. In Fig. 4 the upper band (245 bp) represents amplified double-stranded DNA and the lower band (170 bp) represents amplified single-stranded DNA. Lane 4 indicates that HSV DNA in 1 µg of latent genomic ganglion DNA could be detected, and that the signal obtained was approximately the same as the signal obtained from the amplification of 0.01 ng HSV-1 Bam-B fragment mixed with 1 µg of uninfected ganglion DNA (Lane 6). Since a mouse ganglion contains about 5 µg of DNA, the results of this example show that latent HSV DNA sequences in a signle ganglion can be detected and quantitated.

## Example 4

The methods described in Example 1 can be used to detect and identify the previously uncharacterized known human retroviruses by using the two primers for human retrovirus reverse transcriptase set forth above.

Each sample of RNA extracted from nervous system tissue which is amplified an appropriate number of cycles by the Example 1 methods is subjected to electrophoresis in a 2% agarose gel and the gel is stained with ethidium bromide and photographed. The DNA in the gel is transferred to a Zetaprobe membrane using the alkaline blotting technique, which membrane is sequentially hybridized with each of the $^{32}$P-labelled internal oligonucleotide probes set forth above for HIV, HTLV-I, and HTLV-II and autoradiographed. Autoradiograms resulting from gels hybridized with the HIV, HTLV-I and HTLV-II internal labelled probes showing amplified bands corresponding to the expected approximately 128 base pairs serve to identify (1) the presence of a human retrovirus and (2) the specific human retrovirus depending on which of the three human retrovirus internal probes of this invention hybridized with the amplified product.

The techniques of the present invention can be modified to allow further quantification of samples. In brief, the region targeted for amplification is altered in length and composition by insertion of a short oligonucleotide, which yields a control RNA or DNA template. The control template is included in the experimental RNA or DNA samples in a fixed concentration. The internal oligonucleotide probe used to identify the altered sequence is different from that used for the experimental samples to allow the distinction between amplification product from the control template contained in the same mixture as the experimental unknown template. Since the amount of control template is constant, the determination of the ratio of amplification of the control versus the experimental unknown template forms the basis of quantification of the experimental sample. It is also possible to include oligonucleotide primers specific for the β-actin gene in test reactions to provide a positive control for the integrity of the RNA or DNA template and also to allow for relative quantitation of any viral sequence which is amplified.

Amplification products obtained by the methods of the present invention can also be identified by sequencing the amplification product. The amplification reaction is terminated by the addition of EDTA to 20 mM final concentration. The reaction mixture is concentrated by ethanol precipitation and electrophoresed in a 4% "Nu Sieve" (FMC Bioproducts) agarose gel together with 1 µg of ΦX174 DNA cleaved with HaeIII to serve as a size marker. The amplified DNA band of the correct size is excised from the gel and purified using the phenol-freeze thaw technique. About 0.20-0.25 pmole of the amplified target sequence is then mixed with 2.0-2.5 pmole of $^{32}$P-labelled sequencing primer in 20 µl 70 mM Tris-HCl pH 7.6, 50 mM NaCl, 50 mM β-mercaptoethanol, 50 mM MgCl$_2$ and 0.2 mM EDTA. The probe primer is also used as a sequencing primer. This is possible since it has been located asymmetrically at one end of the amplified target sequence to maximize the sequence information that can be obtained. The dideoxy sequencing reaction is carried out essentially as described by Wrischnik, et al., Nucl.Acid Res. 15:529-542 (1987). The nucleotide sequence is obtained and compared to the known viral sequences.

**Claims**

1. A method of detecting and identifying a human retrovirus in a human blood or tissue sample, comprising:
amplifying a portion of the human retrovirus reverse transcriptase RNA present in said sample;
hybridizing an internal oligonucleotide probe complementary to at least a portion of the amplification product; and
identifying the human retrovirus present in said sample by hybridization with at least one internal probe known to be complementary to at least a portion of the amplification products of known human retroviruses.

2. The method of claim 1, wherein said human retrovirus reverse transcriptase is amplified by adding to a sample of human retrovirus reverse transcriptase RNA two converging oligonucleotide primers at least one of which is complementary to a sequence included in said RNA and at least one of said primers is thereafter extended with one or both of reverse transcriptase and a DNA polymerase such that repeated extentions of said primers yields copies of the sequence between said two primers.

3. The method of claim 1, wherein said reverse transcriptase RNA is amplified by:
annealing first and second converging primers complementary to different portions of said RNA;
extending said first primer with reverse transcriptase to produce a DNA transcript; and
amplifying said transcript with a DNA polymerase also effective to extend said second primer.

4. The method of claim 2 or 3 wherein said primers are complementary to conserved nuclectide sequences of the human retroviruses.

5. The method of claim 4 wherein said conserved nucleotide sequences of the human retroviruses are from the human retroviral pol gene.

6. The method of claim 2 or 3 wherein said primers comprise:
5'-G G A T C C G T G C T T C C A C A G G G A-3' (2417-2431)
5'-A A G C T T A T C A T C C A T G T A T T G-3' (2533-2519).

7. The method of claim 1 wherein a nucleotide fragment having about 245 base pairs is amplified.

8. The method of claim 1 wherein said internal probe for HIV comprises the nucleotide sequence:
5'-G G A T C A C C A G C A A T A T T C C A-3' (2438-2457).

9. The method of claim 1 wherein said internal probe for HTLV-I comprises the nucleotide sequence:
5'-A A T A G T C C C A C C C T G T T C G A-3' (2970-2989).

10. The method of claim 1 wherein said internal probe for HTVL-II comprises the nucleotide sequence:
5'-A A C A G C C C C A C C C T C T T C G A-3' (2968-2987).

11. A method of detecting and identifying human viral sequences associated with multiple sclerosis in a blood or tissue sample, comprising:
amplifying a portion of RNA or DNA present in said sample;
hybridizing an internal oligonucleotide probe complementary to at least a portion of the amplification product; and
identifying the human viral sequence present in said sample by hybridization with at least one internal probe known to be complementary to at least a portion of the amplification product.

12. The method of claim 11 wherein said human viral sequence comprises the $IE_1$ gene of herpes simplex virus-1 (HSV-1).

13. The method of claim 11 wherein said human viral sequence comprises conserved nucleotide sequence of the pol gene of human retrovirus reverse transcriptase.

14. The method of claim 12 wherein said $IE_1$ viral sequence is amplified using olignoucleotide primers comprising the sequences:
5'-G G A T C C G G G C G T G G A G G G T G G G C A C-3' (2966-2986) and
5'-A A G C T T C C C T G G C C G C G C C C C C C C G G C C C-3' (3201-3179).

15. The method of claim 14 wherein said internal probe complementary to at least a portion of said $IE_1$-HSV-1 viral sequences comprises:
5'-C T G G C C C G A C C C G C G C C T C T T C C T C-3' (3094-3070).

16. The method of claim 13 wherein said human retrovirus reverse transcriptase is amplified using oligonucleotide primers comprising the sequences:
5'-G G A T C C G T G C T T C C A C A G G G A-3' (2417-2431)
5'-A A G C T T A T C A T C C A T G T A T T G-3' (2533-2519).

17. The method of claim 13 wherein said internal probe complementary to at least a portion of said human retrovirus reverse transcription viral sequence comprises
5'-G G A T C A C C A G C A A T A T T C C A-3' (2438-2457) for HIV;
5'-A A T A G T C C C A C C C T G T T C G A-3' (2970-2989) for HTLV-I; and
5'-A A C A G C C C C A C C C T C T T C G A-3' (2968-2987) for HTLV-II.

FIG. 1

FIG. 2

FIG. 3

FIG.4

245bp

~170bp